(19) Europäisches Patentamt
European Patent Office
Office européen des brevets

(11) **EP 3 739 326 B1**

(12) **EUROPEAN PATENT SPECIFICATION**

(45) Date of publication and mention
of the grant of the patent:
**16.11.2022 Bulletin 2022/46**

(21) Application number: **19174828.4**

(22) Date of filing: **16.05.2019**

(51) International Patent Classification (IPC):
***G01N 27/22*** *(2006.01)*

(52) Cooperative Patent Classification (CPC):
**G01N 27/223;** G01N 33/383; G01N 33/46

(54) **METHOD, SYSTEM AND SENSOR FOR MONITORING AN AREA**

VERFAHREN, SYSTEM UND SENSOR ZUR ÜBERWACHUNG EINES BEREICHS

PROCÉDÉ, SYSTÈME ET CAPTEUR DE SURVEILLANCE D'UNE ZONE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**18.11.2020 Bulletin 2020/47**

(73) Proprietor: **Technische Universität Graz**
**8010 Graz (AT)**

(72) Inventors:
• **NEUMAYER, Markus**
**8010 Graz (AT)**
• **BRETTERKLIEBER, Thomas**
**8010 Graz (AT)**
• **FLATSCHER, Matthias**
**8020 Graz (AT)**

(74) Representative: **Weiser & Voith**
**Patentanwälte Partnerschaft**
**Kopfgasse 7**
**1130 Wien (AT)**

(56) References cited:
**US-A1- 2014 015 329**

• **CONG ZHANG ET AL: "Design of LC-type passive
wireless multi-parameter sensor", NANO/MICRO
ENGINEERED AND MOLECULAR SYSTEMS
(NEMS), 2013 8TH IEEE INTERNATIONAL
CONFERENCE ON, IEEE, 7 April 2013
(2013-04-07), pages 256-259, XP032436706, DOI:
10.1109/NEMS.2013.6559727 ISBN:
978-1-4673-6351-8**
• **Y.CHEN, S.MUTUKUTLA, P.PASUPATHY
D.P.NEIKIRK, S.L.WOOD: "Magneto-inductive
waveguide as a passive wireless sensor net for
structural health monitoring", SPIE, PO BOX 10
BELLINGHAM WA 98227-0010, USA, vol. 7647, 49,
31 December 2010 (2010-12-31), XP040533989,**

**Description**

The present invention relates to a method and a system of monitoring an area for a change in an environmental parameter, comprising at least two passive LC sensors each of which have a coil and a capacitor connected to the coil, which capacitor is sensitive to the environmental parameter. The invention further relates to a passive LC sensor for use in such a method or system.

Different types of LC sensors are known in the art for sensing different environmental parameters, respectively, e.g., pH values, humidity, temperature, strain, etc., by a change in the capacitance of the LC sensor and, consequently, a change in the resonance frequency. For example, C. Zhang et al., Design of LC-type Passive Wireless Multi-parameter Sensor, 8th IEEE International Conference on Nano/Micro Engineered and Molecular Systems (NEMS), April 2013, pp. 256 - 259 describe a miniature LC-type passive wireless multi-parameter sensor on a single substrate, wherein each LC-circuit comprises a capacitor sensitive to a different parameter and is read out by a common readout coil on the substrate.

LC sensors sensing humidity are, for example, used to detect moisture in building structures to prevent damages due to corrosion of metal components, dissolution of materials like wood or gypsum, growth of mould or the like. For doing so, usually large areas have to be monitored.

The monitoring of a (large) area for a change in an environmental parameter, however, requires an optimization between sparse measuring using only very few sensors, on the one hand, and the wiring and instrumentation effort when using multiple sensors, on the other hand. In order to reduce the cabling effort, wireless interfaces can be used. Moreover, active sensors, i.e., sensors that require local power supply, are usually unsuitable due to their need for either frequent battery replacement or additional wiring for the electrical power supply. It has, therefore, been proposed to use passive RFID sensors that are power supplied by the reader at each readout, cf. C. Strangfeld et al., Embedded Passive RFID-based Sensors for Moisture Monitoring in Concrete, IEEE Sensors, Oct. 2017, pp. 1 - 3. When using low frequency RFID, the magnetic coupling merely allows for very limited range readout, such that the reader has to be moved from one to the next sensor. High frequency ("HF") and particularly ultra-high frequency ("UHF") RFID systems are known to provide extended ranges, whereas their high frequency radio waves usually suffer from substantial attenuation effects by the structures that are to be monitored.

In A. Babu et al., An Efficient Readout Scheme for Simultaneous Measurement from Multiple Wireless Passive LC Sensors, IEEE Transactions on Instrumentation and Measurement, vol. 6 67, no. 5, pp. 1161 - 1168, May 2018, it has been proposed to use a specialised readout device comprising a charged capacitor and a readout coil, over which the capacitor is discharged. The readout coil is inductively coupled to the coil of each of a number of LC sensors and, during discharge of the capacitor, stimulates the LC sensors. Each LC sensor has its own unique resonance frequency, such that the change in the environmental parameter is detected in the readout device by a shift in one of the resonance frequencies and correlated with the respective LC sensor. As the readout circuit is inductively coupled to each LC sensor separately and each LC sensor differs in its resonance frequency from all other sensors, this readout scheme cannot be applied to a multiplicity of sensors for monitoring a larger area.

It is an object of the present invention to provide an improved method, system and sensor which allow for a large sensing area and for a long lifetime.

In a first aspect, this object is achieved with a method of monitoring an area for a change in an environmental parameter, comprising:

   providing at least two passive LC sensors each having a coil and a capacitor connected to the coil, which capacitor is sensitive to the environmental parameter;
   placing each of the at least two sensors at a different location in said area such that an array of sensors is formed in which each two adjacent sensors are inductively coupled via their coils;
   feeding an excitation signal into the array at a first sensor of the array and measuring a system response of the array at said first or a second sensor of the array;
   determining a deviation of the system response from a reference response; and
   detecting a change in the environmental parameter when the deviation exceeds a predetermined threshold.

This method can be used for monitoring a large area while requiring only a single connection to one sensor of the array of sensors for excitation and/or measuring and no further wiring or battery supply. Moreover, the method can be applied to a variety of different environmental parameters by using different types of LC sensors, respectively. As such sensors are generally robust they are applicable to harsh environments and offer a long lifetime without any battery change. Due the inductive coupling between adjacent sensors, a direct accessibility of each sensor is not required, such that the sensors can be integrated, e.g., in a building's wall, floor or other structure. Moreover, all sensors may optionally, though not necessarily, have one and the same resonance frequency. Hence, a limitation of the number of sensors in the area due to limited availability of sensors with sufficiently different resonance frequencies does not occur.

It shall be noted that in Y. Chen et al., Magneto-inductive Waveguide as a Passive Wireless Sensor Net for Structural Health Monitoring, Proc. of SPIE, Vol. 7647, 2010, a corrosion detection system is described which comprises corrosion sensor rings that are coaxially arranged to form a one-dimensional magneto-inductive waveguide and have a coil, a capacitor and a wire portion that is exposed to the structure to be monitored and breaks under corrosion such that, when an exposed wire breaks, the waveguide is interrupted and the one-dimensional position of the corrosion can be determined by counting the number of peaks in the passband of the frequency response of the waveguide.

The system response - and the reference response, respectively - can be an impulse or step response or another response to a suitable excitation signal and/or can be used to derive a transfer function therefrom. In an advantageous embodiment of the method, the system response is an impedance of the array measured as an impedance frequency spectrum over a range of frequencies, and the reference response is a reference impedance frequency spectrum. Resonance frequencies can easily be identified and their changes be detected in impedance frequency spectra. In a favourable variant thereof, said deviation is a difference between an impedance value of the impedance frequency spectrum at a frequency within said range and a reference impedance value of the reference impedance frequency spectrum at the same frequency. This is a very straightforward way of determining the deviation.

When a localisation of a detected change in the environmental parameter within the monitored parameter is desired, it is particularly advantageous, when the method comprises, before said step of measuring:

> determining, for each sensor, a frequency of maximum difference in impedance value between the impedance frequency spectrum and the reference impedance frequency spectrum upon a variation in the environmental parameter sensed by the capacitor of that sensor, and
> further comprises, after said step of determining the deviation:

> > determining an actual frequency of maximum difference in impedance value between the impedance frequency spectrum and the reference impedance frequency spectrum, and
> > localising the change in the environmental parameter by identifying the location of that sensor the frequency of maximum difference of which is closest to said actual frequency.

As the location of each sensor in the area is known, the localisation of the change in the environmental parameter is easily possible.

In a beneficial variant, the excitation signal is a chirp signal. The chirp signal can easily be configured to cover the frequency range of the resonance frequencies of the array of sensors in an efficient way.

The reference response may, e.g., be calculated from a mathematical model of the array of sensors. In a favourable embodiment, however, the reference response is a system response which has been measured at said first or said second sensor of the array upon feeding the excitation signal into the array at said first sensor prior to the change in the environmental parameter. Thereby, a realistic reference response is achieved. Moreover, a slight change in the sensors' parameters, e.g., due to aging of the LC sensors, can be accounted for by measuring the reference response anew periodically.

In a particularly advantageous embodiment of the present method said environmental parameter is humidity. Thereby, moisture in buildings or other structure, which is considered as important reason for the deterioration of structural materials, can be detected.

In a second aspect, the present invention creates a system of monitoring an area for a change in an environmental parameter, comprising at least two passive LC sensors each of which having a coil and a capacitor connected to the coil, which capacitor is sensitive to the environmental parameter, and a monitoring device, wherein

> each sensor is configured to be placed at a different location in said area, all sensors forming an array in which each two adjacent sensors are inductively coupled via their coils, and
> wherein the monitoring device comprises:

> > a measuring unit which is connected to a first sensor of said array or to said first and a second sensor of the array and which is configured to feed an excitation signal into the array at said first sensor and to measure a system response of the array at said first or said second sensor;
> > an evaluation unit which is connected to the measuring unit and has a memory and which is configured to determine a deviation of the system response from a reference response stored in the memory; and
> > a detector which is connected to the evaluation unit and configured to detect a change in the environmental parameter when the determined deviation exceeds a predetermined threshold.

Relating to further embodiments of the system and the advantages thereof, it is referred to the above statements on the present method.

In an advantageous embodiment of the system, said measuring unit is connected to said first and/or second sensor of the array by means of an electrically conducting connection of the coil of the respective sensor. As both the first and the second sensor of the array are inductively coupled to one or more adjacent sensors and, via these, to all sensors of the array, a further separate readout coil is not required.

In a particularly favourable embodiment all sensors in the array are identical. This simplifies the production, handling and placing of the sensors at different locations in the area as no differentiation between sensors is required.

In a third aspect, the present invention creates a passive LC sensor for the aforementioned system, wherein the sensor has a coil and a capacitor connected to the coil, which capacitor is sensitive to the environmental parameter, and wherein the capacitor has interdigitated electrodes and is framed by the coil. Favourably, the coil is a single turn coil. Thereby, a sensor with a simple, yet effective structure is achieved. A major part of the sensor can be utilized by the capacitor to sense the environmental parameter while the coil has maximum size as favourable for inductive coupling.

Relating to further embodiments of the sensor and the advantages thereof, it is referred to the above statements on the present method and system.

The invention will now be described in further detail by means of exemplary embodiments thereof under reference to the enclosed drawings, in which:

Fig. 1 shows a system according to the present invention in a schematic overview;
Fig. 2 shows a method to be performed in the system of Fig. 1 in a flowchart;
Figs. 3a and 3b show LC sensors of the system of Fig. 1 in respective equivalent circuit diagrams;
Fig. 4 shows an impedance of the array of sensors of Fig. 1 over a range of frequencies in an impedance frequency spectrum diagram;
Fig. 5 shows an alternative embodiment and layout of the system of the present invention in a schematic overview; and
Fig. 6 shows an LC sensor of the systems of Figs. 1 or 5 in a top view.

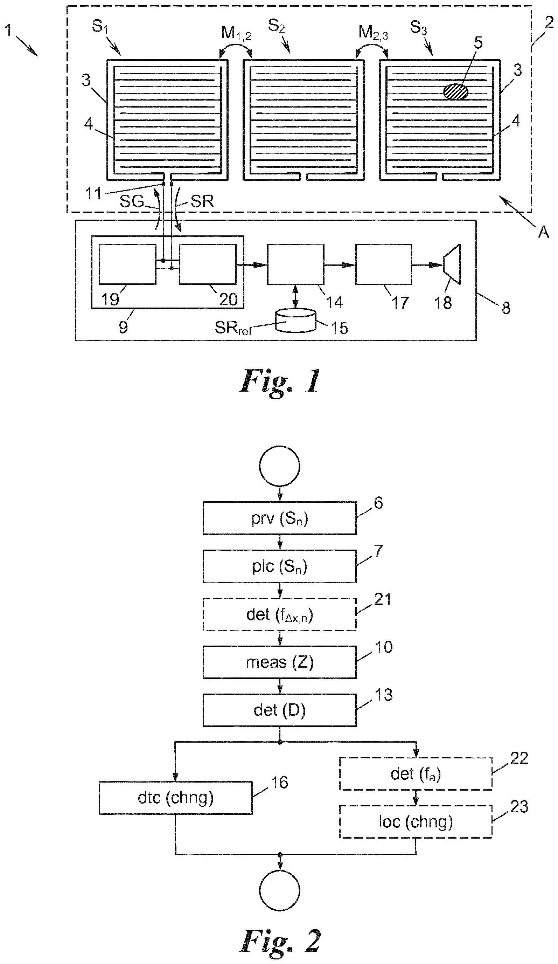

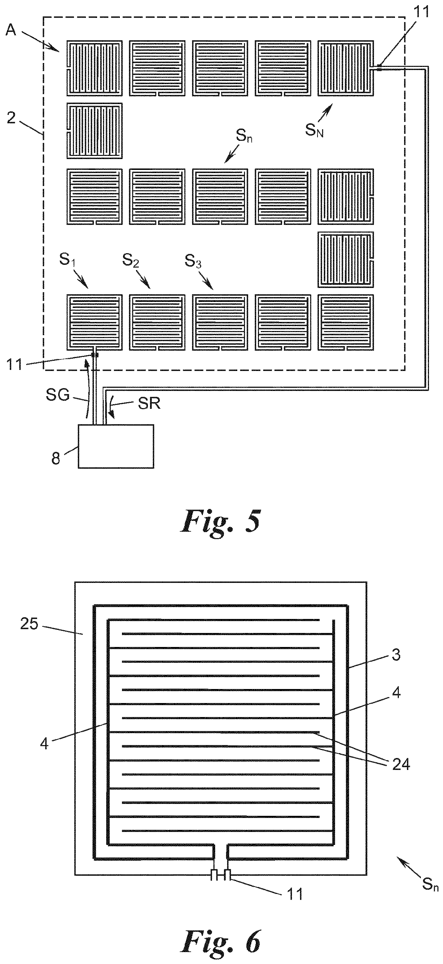

Figs. 1 and 2 show an optional embodiment of a system 1 and method of monitoring an area 2 for a change in an environmental parameter. The system 1 comprises at least two (in the example of Fig. 1: three) passive LC sensors $S_1$, $S_2$, ..., $S_N$, generally $S_n$, N being the number of sensors $S_n$. In the present context, the term "passive" denotes sensors $S_n$ that are not connected to a power supply or a battery. Each sensor $S_n$ has a coil 3 and a capacitor 4 which is connected to the coil 3 as will be explicated in greater detail later with reference to Fig. 6. The capacitor 4 is sensitive to the environmental parameter, which, in the example of Fig. 1, is humidity symbol-ised by a drop of water 5 on the rightmost sensor $S_3$. The term "sensitive" denotes a dependence of the capacitor's capacitance on a change in the environmental parameter.

In a first step 6 of the method (Fig. 2), the passive LC sensors $S_n$ are provided. In subsequent step 7, each of the sensors $S_n$ is placed at a different location in said area 2. Thereby, an array A of sensors $S_n$ is formed, in which array A each two adjacent sensors $S_n$ are inductively coupled via their coils 3, i.e., each pair of two adjacent sensors $S_1$ and $S_2$, $S_2$ and $S_3$, ..., generally $S_m$ and $S_n$, is coupled by individual mutual inductances $M_{1,2}$, $M_{2,3}$, ..., generally $M_{m,n}$.

The area 2 is, e.g., a wall or floor of a building. By monitoring the area 2 for a change in humidity, water or, generally, moisture can be detected, such that a damage due to corrosion of metal components, dissolution of materials like wood or gypsum and/or the growth of mould etc. is discovered or prevented. In other embodiments, the capacitor 4 may be sensitive to pH value, temperature, strain, etc., as known in the art. Moreover, the sensors $S_n$ can be used to monitor an area 2 other than in a building, e.g., a reservoir, soil, etc.

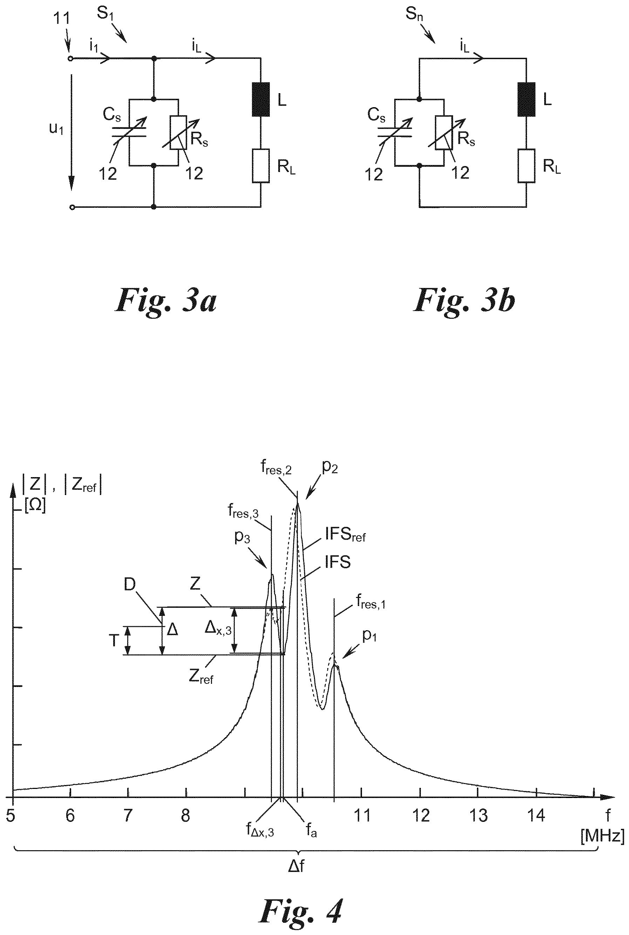

The system 1 further comprises a monitoring device 8 with a measuring unit 9 that is, in the embodiment of Figs. 1 and 2 connected to a first (here: the leftmost) sensor $S_1$ of said array A. In this embodiment, the measuring unit 9 measures, in step 10, an impedance Z of the array A of sensors $S_n$ over a range $\Delta f$ of frequencies f as an impedance frequency spectrum IFS (Fig. 4). To this end, the measuring unit 9 is connected to the first sensor $S_1$ of the array A by means of an electrically conducting connection 11 of the coil 3 of said first sensor $S_1$. Alternatively, the measuring unit 9 could be connected, e.g., via an own readout coil which is inductively coupled to the coil 3 of said first sensor $S_1$.

The principle of said measuring step 10 shall now be explicated on the basis of the equivalent circuit diagrams of Figs. 3a and 3b.

Fig. 3a shows an equivalent circuit model of said first sensor $S_1$ with the electrically conducting connection 11. Fig. 3b shows an equivalent circuit model of another sensor $S_n$ of the array A. The coil 3 of each sensor $S_n$ is modelled by an inductance L and a serial resistor $R_L$ for the ohmic losses. The sensing capacitor 4, on the other hand, is modelled by a capacitance $C_s$ and a parallel resistor $R_s$ which are variable in dependence on the environmental parameter as indicated by arrows 12. Each sensor $S_n$ forms a resonance circuit with an approximate resonance frequency $f_{res}$ according to:

$$f_{res} = \frac{1}{2\pi\sqrt{LC_s}} \cdot \qquad \text{(eq. 1)}$$

To model the interaction between the sensors $S_n$, a state space model shall be considered. Therein, a current $i_1$ is the input quantity (Fig. 3a). The input voltage $u_1$ for the first sensor $S_1$ is given by:

$$u_1 = L\dot{i}_{L,1} + u_{ind,1} + R_{L,1}i_{L,1} , \qquad \text{(eq. 2)}$$

with a mutual induction voltage $u_{ind,1}$ according to:

$$u_{ind,1} = \sum_{n=2}^{N} M_{1,n}\dot{i}_{L,n} \qquad \text{(eq. 3)}$$

with the superscript "**·**" denoting a time derivative,

$i_{L,n}$     being the coil current of another sensor $S_n$, and
$M_{1,n}$    being the individual mutual inductance $M_{m,n}$ between said first sensor $S_1$ and said other sensor $S_n$.

The third equation of the model is

$$C_s\dot{u}_1 + G_su_1 = i_1 - i_{L,1} . \qquad \text{(eq. 4)}$$

Equations (2) to (4) can be formed for the remaining sensors $S_n$ with $i_n = 0$, thus achieving, in matrix-vector notation

$$\mathbf{u} = \mathbf{L}\dot{\mathbf{i}}_L + \mathbf{R}_L\mathbf{i}_L \qquad \text{(eq. 5)}$$

$$\mathbf{C}_s\dot{\mathbf{u}} = \mathbf{e}_1 i_1 - \mathbf{i}_L - \mathbf{G}_s\mathbf{u} \qquad \text{(eq. 6)}$$

with

**L**     being a matrix holding the self and mutual inductances L, $M_{m,n}$,
$\mathbf{C}_s$    being a diagonal matrix of the individual capacitances $C_s$,
$\mathbf{G}_s$    being a diagonal matrix holding the individual conductances $G_s$ of the resistors $R_s$, and
$\mathbf{e}_1$     being the first unit vector.

Therefrom, the state space model of the array A of sensors $S_n$ is derived (eq. 7) and simplified to (eq. 8):

$$\begin{bmatrix} \dot{\mathbf{i}}_L \\ \dot{\mathbf{u}} \end{bmatrix} = \begin{bmatrix} -\mathbf{L}^{-1}\mathbf{R}_L & \mathbf{L}^{-1} \\ -\mathbf{C}_s^{-1} & -\mathbf{C}_s^{-1}\mathbf{G}_s \end{bmatrix}\begin{bmatrix} \mathbf{i}_L \\ \mathbf{u} \end{bmatrix} + \begin{bmatrix} \mathbf{0} \\ \mathbf{C}_s^{-1} \end{bmatrix}\mathbf{e}_1 i_1 \qquad \text{(eq. 7)}$$

$$\dot{\mathbf{x}} = \mathbf{A} \cdot \mathbf{x} + \mathbf{b} \cdot i_1 \qquad \text{(eq. 8)}$$

Moreover, the input voltage $u_1$ for sensor $S_1$ can be expressed by

$$u_1 = \begin{bmatrix} \mathbf{0}^T & \mathbf{e}_1^T \end{bmatrix}\mathbf{x} = \mathbf{c}^T\mathbf{x} \qquad \text{(eq. 9)}$$

which completes the state space model. Hence, using the state space model, the impedance Z of the array A measured at said first sensor $S_1$ is given by

$$\underline{Z}(s) = \frac{\underline{U}_1}{\underline{I}_1} = \mathbf{c}^T \left( s\mathbf{E} - \mathbf{A} \right)^{-1} \mathbf{b} \ . \qquad\qquad (\text{eq. 10})$$

Due to the inductive coupling, information about any sensor $S_n$ in the array A can be obtained at said first sensor $S_1$. The model could be expanded to use any sensor $S_n$ of the array A as the one which is connected to the measuring unit 9 for measuring the impedance Z of the array A. However, in the present context, the principle is explained on the basis of said first sensor $S_1$ of the array A.

Getting back to the example of Figs. 1, 2 and 4, in step 13 a deviation D of the impedance frequency spectrum IFS from a reference impedance frequency spectrum $IFS_{ref}$ is determined by an evaluation unit 14 of the monitoring device 8. To this end, the evaluation unit 14 is connected to the measuring unit 9 and has a memory 15 which stores the reference impedance frequency spectrum $IFS_{ref}$.

In subsequent step 16, a change in the environmental parameter is detected by a detector 17 when the deviation D exceeds a predetermined threshold T. The detector 17 is part of the monitoring device 8 and connected to the evaluation unit 14. As shown in the example of Fig. 1, the monitoring device 8 optionally comprises a notification unit 18, e.g., a display or acoustic alarm, which is connected to the detector 17 and configured to notify the detection of a change in the environmental parameter.

It shall be understood that at least one of the measuring unit 9, the evaluation unit 14, the memory 15 and the detector 17 may be a separate hardware unit, and that the measuring unit 9 may comprise a signal generator 19 for feeding an excitation signal SG into said first sensor $S_1$ and a separate response acquisition unit 20 for capturing the system response SR of the array A in the measuring step 10. Alternatively, at least some of said units 9, 14, 17, 18, 19, 20 may be formed by software blocks in the monitoring device 8.

While the present method and system 1 have been described above on the basis of the specific embodiment of Figs. 1, 2 and 4, they are not limited to measuring, in step 10, an impedance Z and an impedance frequency spectrum IFS and to determining, in step 13, its deviation D from the reference impedance frequency spectrum $IFS_{ref}$. The deviation D can rather more generally be determined in step 13 by the evaluation unit 14 as a system response SR deviating from a reference response $SR_{ref}$ stored in the memory 15. The system response SR, in this general case, is determined in step 10 by the measuring unit 9 which feeds the excitation signal SG into the array A at said first sensor $S_1$, e.g., by means of the signal generator 19, and measures the system response SR which is, e.g., captured by means of the response acquisition unit 20 at said first sensor $S_1$ or at a second sensor $S_N$ as will be explained later.

Apart from the exemplary impedance frequency spectrum IFS, the system response SR may, e.g., be an impulse or step response. Also a transfer function may optionally be derived from the system response SR, e.g., by means of a network analyser function of the measuring unit 9. In each case, the reference response $SR_{ref}$ is also, e.g., an impulse or step response or a transfer function, respectively, i.e., is of the same type as the system response SR.

On the basis of the examples of Figs. 2 and 4, optional variants of and add-ons to the detecting step 16 shall now be explained.

The diagram of Fig. 4 shows the impedance Z (here: the absolute value of the impedance Z) over a range $\Delta f$ of frequencies f (here: the range between 5 and 15 MHz), in which the individual resonance frequencies $f_{res,n}$ of the sensors $S_n$ are comprised. The solid-line curve in the diagram of Fig. 4 is an exemplary reference impedance frequency spectrum $IFS_{ref}$ for the system 1 in Fig. 1, which has three peaks $p_1$, $p_2$, $p_3$ at the resonance frequencies $f_{res,1}$, $f_{res,2}$, $f_{res,3}$ of the three exemplary sensors $S_1$, $S_2$, $S_3$. Due to the mutual inductances $M_{m,n}$ between the sensors $S_n$, the peaks $p_n$ occur at different resonance frequencies $f_{res,n}$ even when all sensors $S_n$ in the array A are identical, as is optionally the case. Consequently, each peak $p_n$ at its respective resonance frequency $f_{res,n}$ can be attributed to a specific one of the sensors $S_n$, e.g., the rightmost peak $p_1$ to sensor $S_1$, the middle peak $p_2$ to sensor $S_2$ and the leftmost peak $p_3$ to sensor $S_3$ for the example of Fig. 1.

The second, dashed-line curve in Fig. 4 is an impedance frequency spectrum IFS measured in step 10 when, e.g., only the sensor $S_3$ (the rightmost sensor in the example of Fig. 1) is affected by humidity, e.g., the drop of water 5. This is detected by a deviation D of the impedance frequency spectrum IFS from the reference impedance frequency spectrum $IFS_{ref}$. In the example of Fig. 4, the deviation D is a difference $\Delta$ between the impedance value Z (here: the absolute value of the impedance), of the impedance frequency spectrum IFS at a frequency f within said range $\Delta f$ and a reference impedance value $Z_{ref}$ (here: an absolute value of the impedance) of the reference impedance spectrum $IFS_{ref}$ at the same frequency f.

It shall be understood that said deviation D can alternatively be determined in a different way, e.g., by considering phase differences of the complex valued impedances Z and $Z_{ref}$, a standard deviation between the impedance frequency spectrum IFS and the reference impedance spectrum $IFS_{ref}$, or the like.

In an optional embodiment, in step 21 before said measuring step 10, a frequency $f_{\Delta x,1}$, $f_{\Delta x,2}$, ..., generally $f_{\Delta x,n}$,

of maximum difference $\Delta_{x,1}$, $\Delta_{x,2}$, ..., generally $\Delta_{x,n}$, in impedance value Z between the impedance frequency spectrum IFS and the reference impedance frequency spectrum $IFS_{ref}$ is determined for each sensor $S_n$ in the array A upon a variation in the environmental parameter sensed by the capacitor 4 of that sensor $S_n$. The frequency $f_{\Delta x,n}$ determined for each sensor $S_n$, respectively, is stored in said memory 15. After said step 13 of determining the deviation D and prior to, after or even in parallel to step 16 of detecting a change, steps 22 and 23 are performed. In step 22, an actual frequency $f_a$ of maximum difference $\Delta$ in impedance value Z between the impedance frequency spectrum IFS and the reference impedance frequency spectrum $IFS_{ref}$ is determined, e.g., by the evaluation unit 14. In subsequent step 23, the change in the environmental parameter is localised in the monitoring device 8, e.g., by the detector 17 or another unit. To this end, the monitoring device 8 identifies the location of that sensor $S_3$ the frequency $f_{\Delta x,3}$ of maximum difference $\Delta_{x,3}$ of which is closest said actual frequency $f_a$.

It shall be noted that neither the actual frequency $f_a$ nor the determined frequency $f_{\Delta x,n}$ of maximum difference $\Delta_{x,n}$ in impedance value Z are necessarily identical to the resonance frequency $f_{res,n}$ of the peak $p_n$ of the respective sensor $S_n$. It shall further be noted that the location of each sensor $S_n$ in said area 2 is known in the system 1, e.g., stored in said memory 15.

The excitation signal SG which is fed into the array A via the connection 11 at said first sensor $S_1$ in step 10 may be a broadband or multi-frequency signal, e.g., an impulse or step signal, a white noise signal etc., as known in the art to determine an impulse or step response or a transfer function of the array A. In an optional embodiment, the excitation signal SG is a chirp signal SG.

In a further optional embodiment, the reference impedance frequency spectrum $IFS_{ref}$ (or generally: the reference response $SR_{ref}$) is an impedance frequency spectrum IFS (or generally: a system response SR) which has been measured by the measuring unit 9 at the same first sensor $S_1$ of the array A prior to the change in the environmental parameter.

As depicted in the example of Fig. 5, the present method and system 1 are not limited to only three sensors $S_n$. Fig. 5 shows a configuration of a monitoring device 8 which is connected to a multitude of sensors $S_n$ which are placed at different locations in the area 2 to be monitored and form an array A. In this example, the sensors $S_n$ are placed in a queue, one adjacent to the next one. It would, however, be possible to include branch-offs in the array A of sensors $S_n$ and/or to have an asymmetric placement of sensors $S_n$ and/or an asymmetric area 2 to be monitored.

For instance, in order to perform a "through-measurement" of the array A which is known in the art for determining the transfer function, the measuring unit 9 of the monitoring device 8 optionally feeds the excitation signal SG into said first sensor $S_1$ and measures the system response SR at a second sensor (in the example of Fig. 5: the last sensor in the queue) $S_N$. When, in this case, the reference response $SR_{ref}$ is optionally measured by the measuring unit 9 as a system response SR prior to the change in the environmental parameter, it is correspondingly measured at said second sensor $S_N$ upon excitation at said first sensor $S_1$ of the array A. To this end, the measuring unit 9 is connected to the first and the second sensor $S_1$, $S_N$ by means of a respective electrically conducting connection 11. Alternatively, at least one of the connections 11 can be replaced by an inductive coupling via a respective readout coil of the measuring unit 9.

Fig. 6 shows an example of a passive LC sensor $S_n$ for the system 1. The sensor $S_n$ comprises a capacitor 4 which is framed by the coil 3 of the sensor $S_n$. Thereby, the capacitor 4 occupies nearly the full space inside the coil 3. The coil 3 of the sensor $S_n$ is a single turn coil 3 and, as previously noted, has an optional electrically conducting connection 11 for connecting the measuring unit 9 of the monitoring device 8. Moreover, the capacitor 4 has electrodes 24 that are interdigitated like combs meshing with each other. The sensor $S_n$ is optionally realised by means of conductor traces which are placed on a suitable carrier material, e.g., a printed circuit board 25. Hence, the sensor $S_n$ in this case is essentially planar.

It shall be noted that differently formed sensors $S_n$ may be used, particularly such sensors $S_n$ where the capacitor 4 and the coil 3 lie side by side, the coil 3 has more than just a single turn, and/or the electrodes 24 of the capacitor 4 are not interdigitated.

The present invention is not restricted to the specific embodiments described in detail herein, but encompasses those variants, combinations and modifications thereof that fall within the scope of the appended claims.

## Claims

1. A method of monitoring an area (2) for a change in an environmental parameter, comprising:

   providing (6) at least two passive LC sensors ($S_n$) each having a coil (3) and a capacitor (4) connected to the coil (3), which capacitor (4) is sensitive to the environmental parameter;
   **characterised by**:

   placing (7) each of the at least two sensors ($S_n$) at a different location in said area (2) such that an array

(A) of sensors ($S_n$) is formed in which each two adjacent sensors ($S_n$) are inductively coupled via their coils (3); feeding (10) an excitation signal (SG) into the array (A) at a first sensor ($S_1$) of the array (A) and measuring (10) a system response (SR) of the array (A) at said first or a second sensor ($S_1$, $S_N$) of the array (A) ; determining (13) a deviation (D) of the system response (SR) from a reference response ($SR_{ref}$); and detecting (16) a change in the environmental parameter when the deviation (D) exceeds a predetermined threshold (T).

2. The method of claim 1, wherein the system response (SR) is an impedance (Z) of the array (A) measured as an impedance frequency spectrum (IFS) over a range ($\Delta f$) of frequencies (f), and the reference response ($SR_{ref}$) is a reference impedance frequency spectrum ($IFS_{ref}$), and
wherein said deviation (D) is a difference ($\Delta$) between an impedance value (Z) of the impedance frequency spectrum (IFS) at a frequency ($f_a$) within said range ($\Delta f$) and a reference impedance value ($Z_{ref}$) of the reference impedance frequency spectrum ($IFS_{ref}$) at the same frequency ($f_a$).

3. The method according to claim 2, comprising, before said step of measuring (10):

Determining (21), for each sensor ($S_n$), a frequency ($f_{\Delta x,n}$) of maximum difference ($\Delta_{x,n}$) in impedance value (Z) between the impedance frequency spectrum (IFS) and the reference impedance frequency spectrum ($IFS_{ref}$) upon a variation in the environmental parameter sensed by the capacitor (4) of that sensor ($S_n$), and
further comprising, after said step of determining (13) the deviation (D):

determining (22) an actual frequency ($f_a$) of maximum difference ($\Delta$) in impedance value (Z) between the impedance frequency spectrum (IFS) and the reference impedance frequency spectrum ($IFS_{ref}$), and
localising (23) the change in the environmental parameter by identifying the location of that sensor ($S_3$) the frequency of maximum difference ($f_{\Delta x,3}$) of which is closest to said actual frequency ($f_a$).

4. The method according to any one of claims 1 to 3, wherein the excitation signal (SG) is a chirp signal (SG).

5. The method according to any one of claims 1 to 4, wherein the reference response ($SR_{ref}$) is a system response (SR) which has been measured at said first or said second sensor ($S_1$, $S_N$) of the array (A) upon feeding the excitation signal (SG) into the array (A) at said first sensor ($S_1$) prior to the change in the environmental parameter.

6. The method according to any one of claims 1 to 5, wherein said environmental parameter is humidity.

7. A system of monitoring an area (2) for a change in an environmental parameter, comprising at least two passive LC sensors ($S_n$) each of which having a coil (3) and a capacitor (4) connected to the coil (3), which capacitor (4) is sensitive to the environmental parameter, and a monitoring device (8), **characterised in that**

each sensor ($S_n$) is configured to be placed at a different location in said area (2), all sensors ($S_n$) forming an array (A) in which each two adjacent sensors ($S_n$) are inductively coupled via their coils (3), and
**in that** the monitoring device (8) comprises:

a measuring unit (9) which is connected to a first sensor ($S_1$) of said array (A) or to said first and a second sensor ($S_1$, $S_N$) of the array (A), and which is configured to feed an excitation signal (SG) into the array (A) at said first sensor ($S_1$) and to measure a system response (SR) of the array (A) at said first or said second sensor ($S_1$, $S_N$);
an evaluation unit (14) which is connected to the measuring unit (9) and has a memory (15) and which is configured to determine a deviation (D) of the system response (SR) from a reference response ($SR_{ref}$) stored in the memory (15); and
a detector (17) which is connected to the evaluation unit (14) and configured to detect a change in the environmental parameter when the determined deviation (D) exceeds a predetermined threshold (T).

8. The system according to claim 7, wherein the system response (SR) is an impedance (Z) of the array (A) which the measuring unit (9) configured to measure as an impedance frequency spectrum (IFS) over said range ($\Delta f$) of frequencies (f), and the reference response ($SR_{ref}$) is a reference impedance frequency spectrum ($IFS_{ref}$), and
wherein said deviation (D) is a difference ($\Delta$) between an impedance value (Z) of the impedance frequency spectrum (IFS) at a frequency ($f_a$) within said range ($\Delta f$) and a reference impedance value ($Z_{ref}$) of the reference impedance frequency spectrum ($IFS_{ref}$) at the same frequency ($f_a$).

9. The system according to claim 8, wherein, for each sensor ($S_n$), a frequency ($f_{\Delta x,n}$) of maximum difference ($\Delta_{x,n}$) in impedance value (Z) between the impedance frequency spectrum (IFS) and the reference impedance frequency spectrum ($IFS_{ref}$) upon a variation in the environmental parameter sensed by the capacitor (4) of that sensor ($S_n$) is stored in said memory (15),

wherein the evaluation unit (14) is further configured to determine an actual frequency ($f_a$) of maximum difference ($\Delta$) in impedance value (Z) between the impedance frequency spectrum (IFS) and the reference impedance frequency spectrum ($IFS_{ref}$), and

wherein the monitoring device (8) is further configured to localise the change in the environmental parameter by identifying the location of that sensor ($S_3$) the frequency of maximum difference ($f_{\Delta x,3}$) of which is closest to said actual frequency ($f_a$).

10. The system according to any one of claims 7 to 9, wherein said excitation signal (SG) is a chirp signal (SG).

11. The system according to any one of claims 7 to 10, wherein said measuring unit (9) is connected to said first and/or said second sensor ($S_1$, $S_N$) of the array (A) by means of an electrically conducting connection (11) of the coil (3) of the respective sensor ($S_1$, $S_N$).

12. The system according to any one of claims 7 to 11, wherein all sensors ($S_n$) in the array (A) are identical.

13. A passive LC sensor for a system (1) according to any one of claims 7 to 12, wherein the sensor ($S_n$) has a coil (3) and a capacitor (4) connected to the coil (3), which capacitor (4) is sensitive to the environmental parameter, **characterized in that** the capacitor (4) has interdigitated electrodes (24) and is framed by the coil (3).

14. The sensor according to claim 13, wherein the coil (3) is a single turn coil.

15. The sensor according to claim 13 or 14, wherein the coil (3) has an electrically conducting connection (11) for connecting a measuring unit (9) of the system (1).

16. The sensor according to any one of claims 13 to 15, wherein the capacitor (4) is sensitive to humidity.


**Patentansprüche**

1. Verfahren zum Überwachen einer Zone (2) auf eine Änderung eines Umgebungsparameters, umfassend:

Bereitstellen (6) zumindest zweier passiver LC-Sensoren ($S_n$), die jeweils eine Spule (3) und einen mit der Spule (3) verbundenen Kondensator (4) haben, wobei der Kondensator (4) auf den Umgebungsparameter empfindlich ist;
**gekennzeichnet durch**:

Platzieren (7) jedes der zumindest zwei Sensoren ($S_n$) an einem anderen Ort in der genannten Zone (2), sodass ein Feld (A) von Sensoren ($S_n$) gebildet wird, in dem jeweils zwei benachbarte Sensoren ($S_n$) über ihre Spulen (3) induktiv gekoppelt sind;
Einspeisen (10) eines Erregersignals (SG) in das Feld (A) an einem ersten Sensor ($S_1$) des Feldes (A) und Messen (10) einer Systemantwort (SR) des Feldes (A) an dem genannten ersten oder einem zweiten Sensor ($S_1$, $S_N$) des Feldes (A);
Ermitteln (13) einer Abweichung (D) der Systemantwort (SR) von einer Referenzantwort ($SR_{ref}$); und
Detektieren (16) einer Änderung des Umgebungsparameters, wenn die Abweichung (D) einen vorgegebenen Schwellwert (T) überschreitet.

2. Verfahren nach Anspruch 1, wobei die Systemantwort (SR) eine Impedanz (Z) des Feldes (A) ist, die als Impedanzfrequenzspektrum (IFS) über einen Bereich ($\Delta f$) von Frequenzen (f) gemessen wird, und die Referenzantwort ($SR_{ref}$) ein Referenzimpedanzfrequenzspektrum ($IFS_{ref}$) ist, und

wobei die genannte Abweichung (D) eine Differenz ($\Delta$) zwischen einem Impedanzwert (Z) des Impedanzfrequenzspektrums (IFS) bei einer Frequenz ($f_a$) innerhalb des genannten Bereichs ($\Delta f$) und einem Referenzimpedanzwert ($Z_{ref}$) des Referenzimpedanzfrequenzspektrums ($IFS_{ref}$) bei derselben Frequenz ($f_a$) ist.

3. Verfahren nach Anspruch 2, umfassend vor dem genannten Schritt des Messens (10):

Ermitteln (21) einer Frequenz ($f_{\Delta x,n}$) maximaler Differenz ($\Delta_{x,n}$) des Impedanzwerts (Z) zwischen dem Impedanzfrequenzspektrum (IFS) und dem Referenzimpedanzfrequenzspektrum ($IFS_{ref}$) für jeden Sensor ($S_n$) bei einer Veränderung des vom Kondensator (4) dieses Sensors ($S_n$) erfassten Umgebungsparameters, und ferner umfassend, nach dem genannten Schritt des Bestimmens (13) der Abweichung (D):

Ermitteln (22) einer aktuellen Frequenz ($f_a$) maximaler Differenz ($\Delta$) im Impedanzwert (Z) zwischen dem Impedanzfrequenzspektrum (IFS) und dem Referenzimpedanzfrequenzspektrum ($IFS_{ref}$), und Lokalisieren (23) der Änderung des Umgebungsparameters durch Identifizieren des Ortes jenes Sensors ($S_3$), dessen Frequenz maximaler Differenz ($f_{\Delta x,3}$) der genannten aktuellen Frequenz ($f_a$) am nächsten ist.

4. Verfahren nach einem der Ansprüche 1 bis 3, wobei das Erregungssignal (SG) ein Chirpsignal (SG) ist.

5. Verfahren nach einem der Ansprüche 1 bis 4, wobei die Referenzantwort ($SR_{ref}$) eine Systemantwort (SR) ist, die an dem genannten ersten oder dem genannten zweiten Sensor ($S_1$, $S_N$) des Feldes (A) bei Einspeisen des Erregersignals (SG) in das Feld (A) an dem genannten ersten Sensor ($S_1$) vor der Änderung des Umgebungsparameters gemessen wurde.

6. Verfahren nach einem der Ansprüche 1 bis 5, wobei der Umgebungsparameter Feuchtigkeit ist.

7. System zum Überwachen einer Zone (2) auf eine Änderung eines Umgebungsparameters, umfassend zumindest zwei passive LC-Sensoren ($S_n$), von denen jeder eine Spule (3) und einen mit der Spule (3) verbundenen Kondensator (4) hat, wobei der Kondensator (4) auf den Umgebungsparameter empfindlich ist, und eine Überwachungsvorrichtung (8), **dadurch gekennzeichnet,**

**dass** jeder Sensor ($S_n$) dazu ausgebildet ist, an einem anderen Ort in der genannten Zone (2) platziert zu werden, wobei alle Sensoren ($S_n$) ein Feld (A) bilden, in dem jeweils zwei benachbarte Sensoren ($S_n$) über ihre Spulen (3) induktiv gekoppelt sind, und
**dass** die Überwachungsvorrichtung (8) umfasst:

eine Messeinheit (9), die an einen ersten Sensor ($S_1$) des Feldes (A) oder an den genannten ersten und einen zweiten Sensor ($S_1$, $S_N$) des Feldes (A) angeschlossen und dazu ausgebildet ist, ein Erregersignal (SG) in das Feld (A) an dem genannten ersten Sensor ($S_1$) einzuspeisen und eine Systemantwort (SR) des Feldes (A) an dem genannten ersten oder dem genannten zweiten Sensor ($S_1$, $S_N$) zu messen; eine Auswerteeinheit (14), die an die Messeinheit (9) angeschlossen ist und einen Speicher (15) hat und dazu ausgebildet ist, eine Abweichung (D) der Systemantwort (SR) von einer in dem Speicher (15) gespeicherten Referenzantwort ($SR_{ref}$) zu ermitteln; und einen Detektor (17), der an die Auswerteeinheit (14) angeschlossen und dazu ausgebildet ist, eine Veränderung des Umgebungsparameters zu detektieren, wenn die ermittelte Abweichung (D) einen vorgegebenen Schwellwert (T) überschreitet.

8. System nach Anspruch 7, wobei die Systemantwort (SR) eine Impedanz (Z) des Feldes (A) ist, welche die Messeinheit (9) als ein Impedanzfrequenzspektrum (IFS) über einen Bereich ($\Delta f$) von Frequenzen (f) zu messen ausgebildet ist, und die Referenzantwort ($SR_{ref}$) ein Referenzimpedanzfrequenzspektrum ($IFS_{ref}$) ist, und wobei die genannte Abweichung (D) eine Differenz ($\Delta$) zwischen einem Impedanzwert (Z) des Impedanzfrequenzspektrums (IFS) bei einer Frequenz ($f_a$) innerhalb des genannten Bereichs ($\Delta f$) und einem Referenzimpedanzwert ($Z_{ref}$) des Referenzimpedanzfrequenzspektrums ($IFS_{ref}$) bei derselben Frequenz ($f_a$) ist.

9. System nach Anspruch 8, wobei für jeden Sensor ($S_n$) eine Frequenz ($f_{\Delta x,n}$) maximaler Differenz ($\Delta_{x,n}$) im Impedanzwert (Z) zwischen dem Impedanzfrequenzspektrum (IFS) und dem Referenzimpedanzfrequenzspektrum ($IFS_{ref}$) bei einer Veränderung des vom Kondensator (4) dieses Sensors ($S_n$) erfassten Umgebungsparameters im genannten Speicher (15) gespeichert ist,

wobei die Auswerteeinheit (14) ferner dazu ausgebildet ist, eine aktuelle Frequenz ($f_a$) maximaler Differenz ($\Delta$) im Impedanzwert (Z) zwischen dem Impedanzfrequenzspektrum (IFS) und dem Referenzimpedanzfrequenzspektrum ($IFS_{ref}$) zu ermitteln, und wobei die Überwachungsvorrichtung (8) ferner dazu ausgebildet ist, die Änderung des Umgebungsparameters durch Identifizieren des Ortes jenes Sensors ($S_3$) zu lokalisieren, dessen Frequenz maximaler Differenz ($f_{\Delta x,3}$) der genannten aktuellen Frequenz ($f_a$) am nächsten ist.

10. System nach einem der Ansprüche 7 bis 9, wobei das Erregersignal (SG) ein Chirpsignal (SG) ist.

11. System nach einem der Ansprüche 7 bis 10, wobei die genannte Messeinheit (9) an den genannten ersten und/oder den genannten zweiten Sensor ($S_1$, $S_N$) des Feldes (A) mithilfe einer elektrisch leitenden Verbindung (11) der Spule (3) des jeweiligen Sensors ($S_1$, $S_N$) angeschlossen ist.

12. System nach einem der Ansprüche 7 bis 11, wobei alle Sensoren ($S_n$) in dem Feld (A) identisch sind.

13. Passiver LC-Sensor für ein System (1) nach einem der Ansprüche 7 bis 12, wobei der Sensor ($S_n$) eine Spule (3) und einen mit der Spule (3) verbundenen Kondensator (4) hat, wobei der Kondensator (4) auf den Umgebungsparameter empfindlich ist, **dadurch gekennzeichnet, dass** der Kondensator (4) Interdigitalelektroden (24) hat und von der Spule (3) eingerahmt ist.

14. Sensor nach Anspruch 13, wobei die Spule (3) eine Einzelwindungsspule ist.

15. Sensor nach Anspruch 13 oder 14, wobei die Spule (3) eine elektrisch leitende Verbindung (11) zum Verbinden einer Messeinheit (9) des Systems (1) hat.

16. Sensor nach einem der Ansprüche 13 bis 15, wobei der Kondensator (4) auf Feuchtigkeit empfindlich ist.

**Revendications**

1. Procédé de surveillance d'une zone (2) pour un changement dans un paramètre environnemental, comprenant :

   la fourniture (6) d'au moins deux capteurs ($S_n$) LC passifs ayant chacun une bobine (3) et un condensateur (4) connecté à la bobine (3), lequel condensateur (4) est sensible au paramètre environnemental ;
   **caractérisé par** :

   la mise en place (7) de chacun des au moins deux capteurs ($S_n$) à un endroit différent dans ladite zone (2) de sorte qu'un réseau (A) de capteurs ($S_n$) soit formé, dans lequel chaques deux capteurs ($S_n$) adjacents sont couplés par induction par le biais de leurs bobines (3) ;
   l'injection (10) d'un signal d'excitation (SG) dans le réseau (A) au niveau d'un premier capteur ($S_1$) du réseau (A) et la mesure (10) d'une réponse système (SR) du réseau (A) au niveau dudit premier ou d'un deuxième capteur ($S_1$, $S_N$) du réseau (A) ;
   la détermination (13) d'une déviation (D) de la réponse système (SR) par rapport à une réponse de référence ($SR_{ref}$) ; et
   la détection (16) d'un changement du paramètre environnemental lorsque la déviation (D) dépasse un seuil (T) prédéterminé.

2. Procédé selon la revendication 1, dans lequel la réponse système (SR) est une impédance (Z) du réseau (A) mesurée sous forme d'un spectre de fréquences d'impédance (IFS) sur une plage ($\Delta f$) de fréquences (f), et la réponse de référence ($SR_{ref}$) est un spectre de fréquences d'impédance de référence ($IFS_{ref}$), et
   dans lequel ladite déviation (D) est une différence ($\Delta$) entre une valeur d'impédance (Z) du spectre de fréquences d'impédance (IFS) à une fréquence ($f_a$) dans ladite plage ($\Delta f$) et une valeur d'impédance de référence ($Z_{ref}$) du spectre de fréquences d'impédance de référence ($IFS_{ref}$) à la même fréquence ($f_a$).

3. Procédé selon la revendication 2, comprenant, avant ladite étape de mesure (10) :

   la détermination (21), pour chaque capteur ($S_n$), d'une fréquence ($f_{\Delta x,n}$) de différence maximale ($\Delta_{x,n}$) dans la valeur d'impédance (Z) entre le spectre de fréquences d'impédance (IFS) et le spectre de fréquences d'impédance de référence ($IFS_{ref}$) lors d'une variation dans le paramètre environnemental détecté par le condensateur (4) de ce capteur ($S_n$), et
   comprenant en outre, après ladite étape de détermination (13) de la déviation (D) :

la détermination (22) d'une fréquence actuelle ($f_a$) de différence maximale ($\Delta$) dans la valeur d'impédance (Z) entre le spectre de fréquences d'impédance (IFS) et le spectre de fréquences d'impédance de référence ($IFS_{ref}$), et

la localisation (23) du changement dans le paramètre environnemental en identifiant la position de ce capteur ($S_3$) dont la fréquence de différence maximale ($f_{\Delta x,3}$) est la plus proche par rapport à ladite fréquence actuelle ($f_a$).

4. Procédé selon l'une quelconque des revendications 1 à 3, dans lequel le signal d'excitation (SG) est un signal chirp (SG) .

5. Procédé selon l'une quelconque des revendications 1 à 4, dans lequel la réponse de référence ($SR_{ref}$) est une réponse système (SR) qui a été mesurée au niveau dudit premier ou dudit deuxième capteur ($S_1$, $S_N$) du réseau (A) lors de l'injection du signal d'excitation (SG) dans le réseau (A) au niveau dudit premier capteur ($S_1$) avant le changement dans le paramètre environnemental.

6. Procédé selon l'une quelconque des revendications 1 à 5, dans lequel ledit paramètre environnemental est l'humidité.

7. Système de surveillance d'une zone (2) pour un changement dans un paramètre environnemental, comprenant au moins deux capteurs ($S_n$) LC passifs dont chacun possède une bobine (3) et un condensateur (4) connecté à la bobine (3), lequel condensateur (4) est sensible au paramètre environnemental, et un dispositif de surveillance (8), **caractérisé en ce que**

chaque capteur ($S_n$) est conçu pour être placé à un endroit différent dans ladite zone (2), tous les capteurs ($S_n$) formant un réseau (A) dans lequel chaques deux capteurs ($S_n$) adjacents sont couplés par induction par le biais de leurs bobines (3), et

**en ce que** le dispositif de surveillance (8) comprend :

une unité de mesure (9) qui est connectée à un premier capteur ($S_1$) dudit réseau (A) ou audit premier et à un deuxième capteur ($S_1$, $S_N$) du réseau (A), et qui est conçu pour injecter un signal d'excitation (SG) dans le réseau (A) au niveau dudit premier capteur ($S_1$) et pour mesurer une réponse système (SR) du réseau (A) au niveau dudit premier ou dudit deuxième capteur ($S_1$, $S_N$) ;

une unité d'évaluation (14) qui est connectée à l'unité de mesure (9) et possède une mémoire (15), et qui est conçue pour déterminer une déviation (D) de la réponse système (SR) par rapport à une réponse de référence ($SR_{ref}$) stockée dans la mémoire (15) ; et

un détecteur (17) qui est connecté à l'unité d'évaluation (14) et conçu pour détecter un changement dans le paramètre environnemental lorsque la déviation (D) déterminée dépasse un seuil (T) prédéterminé.

8. Système selon la revendication 7, dans lequel la réponse système (SR) est une impédance (Z) du réseau (A) que l'unité de mesure (9) est conçue pour mesurer sous forme d'un spectre de fréquences d'impédance (IFS) sur une plage ($\Delta f$) de fréquences (f), et la réponse de référence ($SR_{ref}$) est un spectre de fréquences d'impédance de référence ($IFS_{ref}$), et

dans lequel ladite déviation (D) est une différence ($\Delta$) entre une valeur d'impédance (Z) du spectre de fréquences d'impédance (IFS) à une fréquence ($f_a$) dans ladite plage ($\Delta f$) et une valeur d'impédance de référence ($Z_{ref}$) du spectre de fréquences d'impédance de référence ($IFS_{ref}$) à la même fréquence ($f_a$).

9. Système selon la revendication 8, dans lequel, pour chaque capteur ($S_n$), une fréquence ($f_{\Delta x,n}$) de différence maximale ($\Delta_{x,n}$) dans la valeur d'impédance (Z) entre le spectre de fréquences d'impédance (IFS) et le spectre de fréquences d'impédance de référence ($IFS_{ref}$) lors d'une variation dans le paramètre environnemental détecté par le condensateur (4) de ce capteur ($S_n$) est stockée dans ladite mémoire (15),

dans lequel l'unité d'évaluation (14) est en outre conçue pour déterminer une fréquence actuelle ($f_a$) de différence maximale ($\Delta$) dans la valeur d'impédance (Z) entre le spectre de fréquences d'impédance (IFS) et le spectre de fréquences d'impédance de référence ($IFS_{ref}$), et

dans lequel le dispositif de surveillance (8) est en outre conçu pour la localisation du changement dans le paramètre environnemental en identifiant la position de ce capteur ($S_3$) dont la fréquence de différence maximale ($f_{\Delta x,3}$) est la plus proche par rapport à ladite fréquence actuelle ($f_a$).

10. Système selon l'une quelconque des revendications 7 à 9, dans lequel le signal d'excitation (SG) est un signal chirp (SG) .

**11.** Système selon l'une quelconque des revendications 7 à 10, dans lequel ladite unité de mesure (9) est connectée audit premier et/ou audit deuxième capteur ($S_1$, $S_N$) du réseau (A) au moyen d'une connexion électriquement conductive (11) de la bobine (3) du capteur ($S_1$, $S_N$) respectif.

**12.** Système selon l'une quelconque des revendications 7 à 11, dans lequel tous les capteurs ($S_n$) dans le réseau (A) sont identiques.

**13.** Capteur LC passif pour un système (1) selon l'une quelconque des revendications 7 à 12, dans lequel le capteur ($S_n$) possède une bobine (3) et un condensateur (4) connecté à la bobine (3), lequel condensateur (4) est sensible au paramètre environnemental, **caractérisé en ce que** le condensateur (4) possède des électrodes (24) interdigitées et est encadré par la bobine (3).

**14.** Capteur selon la revendication 13, dans lequel la bobine (3) est une bobine à un seul tour.

**15.** Capteur selon la revendication 13 ou 14, dans lequel la bobine (3) possède une connexion électriquement conductive (11) pour connecter une unité de mesure (9) du système (1).

**16.** Capteur selon l'une quelconque des revendications 13 à 15, dans lequel le condensateur (4) est sensible à l'humidité.

**Fig. 1**

**Fig. 2**

**Fig. 3a**                    **Fig. 3b**

**Fig. 4**

**Fig. 5**

**Fig. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **C. ZHANG et al.** Design of LC-type Passive Wireless Multi-parameter Sensor. *IEEE International Conference on Nano/Micro Engineered and Molecular Systems (NEMS),* April 2013, 256-259
- **C. STRANGFELD et al.** Embedded Passive RFID-based Sensors for Moisture Monitoring in Concrete. *IEEE Sensors,* October 2017, 1-3

- **A. BABU et al.** An Efficient Readout Scheme for Simultaneous Measurement from Multiple Wireless Passive LC Sensors. *IEEE Transactions on Instrumentation and Measurement,* May 2018, vol. 6 67 (5), 1161-1168
- **Y. CHEN et al.** Magneto-inductive Waveguide as a Passive Wireless Sensor Net for Structural Health Monitoring. *Proc. of SPIE,* 2010, vol. 7647